# EUROPEAN PATENT SPECIFICATION

(11) **EP 3 856 713 B1**
(45) Date of publication and mention of the grant of the patent: **09.11.2022**
(21) Application number: 19769191.8
(22) Date of filing: 19.09.2019
(51) Int. Cl.: C07C 303/06, C07C 309/04

(54) **CATIONS AS CATALYST IN THE PRODUCTION OF ALKANE SULFONIC ACIDS**
KATIONEN ALS KATALYSATOR BEI DER HERSTELLUNG VON ALKANSULFONSÄUREN
CATIONS COMME CATALYSEUR DANS LA PRODUCTION D'ACIDES SULFONIQUES D'ALCANE

(30) Priority: 25.09.2018 EP 18196520
(43) Date of publication of application: 04.08.2021
(73) Proprietor: BASF SE, 67056 Ludwigshafen am Rhein (DE)
(72) Inventor: OTT, Timo, 47169 Duisburg (DE); BIERTUEMPEL, Ingo, 47169 Duisburg (DE)
(74) Representative: BASF IP Association
(86) International application number: PCT/EP2019/075233
(87) International publication number: WO 2020/064515

(56) References cited:
- WO-A1-2018/146153
- SUDIP MUKHOPADHYAY ET AL: "Direct Sulfonation of Methane to Methanesulfonic Acid by Sulfur Trioxide Catalyzed by Cerium(IV) Sulfate in the Presence of Molecular Oxygen", ADVANCED SYNTHESIS & CATALYSIS, vol. 346, no. 8, 1 July 2004 (2004-07-01), pages 913-916, XP055389004, DE ISSN: 1615-4150, DOI: 10.1002/adsc.200404060

## Description

The present invention relates to the use of cations or compounds forming stable cations in the production of alkane sulfonic acids as well as methods to produce methane sulfonic acids employing the cations as catalyst.

Alkane sulfonic acids are organic acids that can reach a similar acid strength as that of inorganic mineral acids, for example, sulfuric acid. However, in contrast to usual mineral acids such as sulfuric and nitric acids, the sulfonic acids are non-oxidizing and do not give off vapors that are harmful to health, as can be observed with hydrochloric and nitric acids. Further, many sulfonic acids, for example, methane sulfonic acid, are biologically degradable. The applications of sulfonic acids are many, for example, in cleaning agents, surfactants, galvanic and electronic industry, as catalysts, and in organic synthesis, pharmaceutical chemistry, for example, as protective groups. The salts of sulfonic acids are employed, for example, as surfactants, for example, sodium dodecylsulfonate, or in the electroplating industry, especially as tin, zinc, silver, lead and indium, but also other metal, alkylsulfonates. Furthermore, organic salts are employed in pharmaceutical chemistry. The very high solubility of alkyl sulfonates plays an important role, in particular. Further, no harmful gases are formed in electrolysis, and the use of toxic compounds, for example, cyanide, which is common in many cases, is dispensed with.

The structurally simplest representative of alkane sulfonic acids is methane sulfonic acid. US 2,493,038 describes the preparation of methane sulfonic acid from SO₃ and methane. US 2005/0070614 describes further methods for preparing methane sulfonic acid, and its application. The methods known in the prior art are in part complicated, cost-intensive, and lead to undesirable products because of the harsh reaction conditions.

The reaction conditions in conventional processes of alkanesulfonic acid production can result in undesirable side products, which even manifest themselves as disturbing inhibitors in the production of alkanesulfonic acids. This may lead to termination of the actual reaction for preparing the alkanesulfonic acid, but also to impurities, formation of side products and poor yields, based on sulfur trioxide and methane.

WO 2007/136425 A2 discloses the use of the compound di(methanesulfonyl) peroxide (DMSP), which must be prepared by a complex electrolysis and, in addition, is a crystallizable highly explosive solid, as an initiator in a reaction in which methanesulfonic acid is formed from sulfur trioxide and methane.

WO 2015/071365 A1 and WO 2015/071455 A1 both describe processes for the sulfonation of alkanes. The main steps are:
Synthesis of an initiator/initiator-solution.
Preparation of a sulfur trioxide-solution (oleum) by dissolving sulfur trioxide in an inert solvent (e.g. sulfuric acid)
Reaction of oleum with the corresponding alkane after or during addition of the initiator/ initiator-solution in a high-pressure-reactor.
Quenching of non-reacted starting material
Purification (e.g. distillation, crystallization etc.)
Recycling of the inert solvent (e.g. sulfuric acid).

According to said prior art, the initiator is particularly prepared by reacting an alkane sulfonic acid R-SO₃H, i.e. the desired product, with hydrogen peroxide to form an initiator-precursor R-SO₂-O-OH. Said initiator-precursor is then reacted with SO₃ yielding initiator compounds such as R-SO₂-O-O-SO₃H. The cited prior art therefore requires some amount of the desired product to form an initiator.

WO 2018/146153 A1 also discloses a method for the production of alkane sulfonic acids. In this process, a carbocation is used, usually a carbenium. It is obtained by reacting an alkane with an activade pre-catalyst, wherein the pre-catalyst comprises a hydrogen peroxided derivative and wherein the pre-catalyst is activated by reacting the pre-catalyst with a super acid.

It is thus the object of the present invention to provide novel catalysts for the homogeneous catalysis in the preparation of alkane sulfonic acids, especially methane sulfonic acid (MSA). Particularly, it is the object of the invention to provide catalysts that do not require the desired product itself to be present as an initiator-precursor. Further, requirements for sulfur trioxide and alkanes should be of no relevance, meaning that not only absolute pure raw materials might be used, but that impurities do not affect negatively the reaction.

In a first embodiment, the object of the present invention is solved by the subject of claim 1.

The alkyl cation will afterwards react with sulfur trioxide forming the alkane sulfonic acid. Particularly, methane, ethane, propane, butane, isopropane, isobutane or a higher alkane can be reacted with sulfur trioxide to form the corresponding alkane sulfonic acid. Higher alkane within the meaning of the present application are straight or branched alkanes with 20 C-atoms or less.

In the present specification, the terms "alkane cation" and "alkyl cation" are used synonymously.

Surprisingly it has been found that cations which are stable under super acid conditions are able to react under said conditions with the alkane ALK and form an alkane cation:

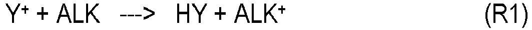

The cation Y⁺ may be either a stable cation and added as cation to the reaction solution. Alternatively, the cation can form *in situ* during the reaction according to the following reaction scheme:

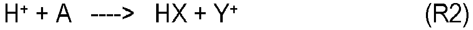

The cation is used in the production of alkane sulfonic acid from an alkane and sulfur trioxide. The reaction conditions are strongly acid or even super acid in his reaction. A super acid is an acid with an acidity greater than that of 100% pure sulfuric acid. Strongly acid means an acidity of 100% pure sulfuric acid or at least similar to it.

If the cation is stable, stability in this context means that it is able to react with the alkane but does not decompose within 24 h at room temperature (20 °C), i.e. the half-life time t_{1/2} at room temperature is at least 24 h, preferably at least 30 h, especially at least 48 h.

Stable cations are formed prior to their use, i.e. prior to their addition into the reactor in which the reaction between alkane and sulfur trioxide takes place. Preferably, one type of cation is used alone and not together with another type of cation.

Alternatively, the cation is produced *in situ* during the production of the alkane sulfonic acid. In such cases a compound is added to the reaction and the cation is formed according to the above shown reaction (R2). Suitable compounds to be used are halogens, especially I₂ and Br₂, inter halogen compounds, especially I-Br, or solid elements of the 15^{th} or 16^{th} group of the periodic table of elements, especially S, Se, Te, P, As, Sb.

If halogens or interhalogens are used as compounds, the bond between the halogens breaks heterolytically. Iodine would thus react to HI and I⁺, bromine to HBr and Br, and the interhalogen either to HI and Br or to HBr and I⁺. The reactive cation would be I⁺ or Br, which is formed *in situ,* and afterwards reacts with the alkane to the alkane cation as schematically shown above in (R1).

If solid elements of the 15^{th} or 16^{th} group of the periodic table are used, they may form oligomeric or polymeric cationic compounds, i.e., sulfur will form an oligomer Sₙ-S⁺, wherein n may for example be in the range of from 0 to 10, preferably from 2 to 10, or in another range. Said Sₙ-S⁺ would be the reactive compound. Similar compounds may also occur with the other elements. Alternatively, they can form cations without polymerisation/oligomerisation. To sum up all possible cations of S, Se, Te, As, Sb and P, they are summarized with S⁺, Se⁺, Te⁺, As⁺, Sb⁺, and P⁺ respectively. Additionally, also Silicon may be added to the reaction solution forming Si+ as cation.

In another embodiment, the object of the present invention is solved by a process for the preparation of alkane sulfonic acids, especially of methane sulfonic acid, comprising the steps of
i) providing sulfur trioxide and an alkane, especially methane, to a reaction chamber,
ii) setting a pressure of from 1 to 200 bar to the reactor,
iii) introducing a compound forming *in situ a* cation into said reactor, wherein the cation is selected from a halogen cation, especially I⁺ and Br, S⁺, Se⁺, Te⁺, As⁺, Sb⁺, P⁺ and/or Si⁺,
iv) controlling the temperature in the reactor to be at 0 °C to 100 °C,
v) after the reaction is finished, if necessary, purifying the reaction product.

In a further embodiment, the object of the present invention is solved by a process for the preparation of alkane sulfonic acids, especially of methane sulfonic acid, comprising the steps of
i) providing sulfur trioxide and an alkane, especially methane, to a reaction chamber,
ii) setting a pressure of from 1 to 200 bar to the reactor,
iii) introducing a cation being stable under super acid conditions into said reactor, wherein stability means that the cation is able to react with the alkane but does not decompose within 24 h at room temperature (20 °C), and wherein the cation is selected from a halogen cation, especially I⁺ and Br, S⁺, Se⁺, Te⁺, As⁺, Sb⁺, P⁺ and/or Si⁺,
iv) controlling the temperature in the reactor to be at 0 °C to 100 °C,
v) after the reaction is finished, if necessary, purifying the reaction product.

The reaction product in both cases is a mixture of alkane sulfonic acid, sulfuric acid and potentially an excess of SO₃. Further, impurities of the catalyst may be used. To obtain the pure alkane sulfonic acid, especially methane sulfonic acid, the reaction product is purified, especially by distillation.

Sulfur trioxide may be provided in the form of oleum, i.e., a solution of sulfur trioxide in sulfuric acid. Instead of oleum also pure sulfur trioxide can be employed. This avoids the preparation of sulfur trioxide solutions. The reaction conditions are here without added solvents. Further, non-reacted sulfur trioxide can evaporate, avoiding the necessity of quenching it.

In a further embodiment, sulfur trioxide is used in a form of oleum with a trioxide content of 50 % (w/w) or less, or 65 % (w/w) or more. Surprisingly it has been found that for the processes of the present invention also oleum with a sulfur trioxide content of 65 % (w/w) or more, especially of 70 % w/w or more can be used without negatively affecting the inventive process. Even pure sulfur trioxide (100 % (w/w) sulfur trioxide) may be used.

The temperature during the reaction is preferably within a range from above 0 °C to 70 °C, especially from 10 °C to 65 °C, preferably from 20 °C to 60 °C. Surprisingly the formation of side products was lower at lower temperatures. If the temperature is around 0 °C or 10 °C, the reaction takes place but needs a longer time so that for an economically process the temperature is preferably 20 °C or above, especially about 40 °C to 55 °C.

The pressure is set to be within a range from 1 to 200 bar, preferably from 50 to 150 bar, especially from 80 to 120 bar.

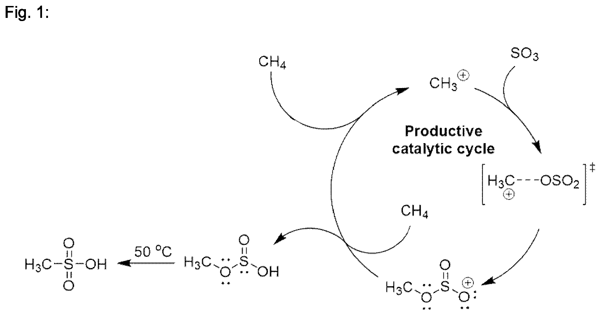

Without being bound to theory, in the process of the invention, the cation formed *in situ or* added in step iii) reacts with the alkane to form an alkane cation, said alkane cation reaction with SO₃ to form an alkane sulfur cation, said alkane sulfur cation reacting again with the alkane to form the alkane sulfonic acid and again an alkane cation. Therefore, the process according to the invention has in a first step the formation of a alkane cation ALK⁺. In case methane is used as alkane, a CH₃⁺ cation is formed which then reacts according to the reaction scheme depicted in fig. 1.

The alkane in the process according to the invention can be any straight chain or branched alkane, preferably with 20 C-atoms or less. Especially preferred, the alkane is selected from methane, ethane, propane, butane, isopropane or isobutane. Accordingly, the respective alkyl sulfonic acid is formed. Preferably, the alkane is methane and the alkyl sulfonic acid is methane sulfonic acid. In this embodiment, the alkane cation is CH₃⁺, as shown in fig. 1.

In another embodiment, the object of the present invention is solved by a mixture comprising an alkane, sulfur trioxide, a cation being able to react with the alkane to form an alkane cation, and optionally a solvent. In an alternative embodiment, the object of the present invention is solved by a mixture comprising an alkane, sulfur trioxide, a compound forming *in situ a* cation under super acid conditions, said cation being able to react with the alkane to form an alkane cation, and optionally a solvent.

Preferably, the solvent is sulfuric acid and/or the alkane it methane.

## Claims

1. Use of a cation being stable under super acid conditions as catalyst in the preparation of alkanes sulfonic acids from alkanes and sulfur trioxide, especially in the preparation of methane sulfonic acid from methane and sulfur trioxide, said cation being able to react with the alkane to form an alkyl cation, wherein stability means that the cation is able to react with the alkane but does not decompose within 24 h at room temperature (20 °C), and wherein the cation is selected from a halogen cation, especially I⁺ and Br⁺, S⁺, Se⁺, Te⁺, As⁺, Sb⁺, P⁺ and/or Si⁺.

2. Use according to claim 1, wherein the cation is formed *in situ* in acid or super acid conditions during the preparation of alkane sulfonic acids.

3. Use according to claim 1, wherein the cation is formed prior to addition to the reaction for obtaining an alkane sulfonic acid.

4. Process for the preparation of alkane sulfonic acids, especially of methane sulfonic acid, comprising the steps of
i) providing sulfur trioxide and an alkane, especially methane, to a reaction chamber,
ii) setting a pressure of from 1 to 200 bar to the reactor,
iii) introducing a compound forming *in situ* a cation into said reactor, wherein the cation is selected from a halogen cation, especially I⁺ and Br⁺, S⁺, Se⁺, Te⁺, As⁺, Sb⁺, P⁺ and/or Si⁺,
iv) controlling the temperature in the reactor to be at 0 °C to 100 °C,
v) after the reaction is finished, if necessary, purifying the reaction product.

5. Process for the preparation of alkane sulfonic acids, especially of methane sulfonic acid, comprising the steps of
i) providing sulfur trioxide and an alkane, especially methane, to a reaction chamber,
ii) setting a pressure of from 1 to 200 bar to the reactor,
iii) introducing a cation being stable under super acid conditions into said reactor, wherein stability means that the cation is able to react with the alkane but does not decompose within 24 h at room temperature (20 °C), and wherein the cation is selected from a halogen cation, especially I⁺ and Br⁺, S⁺, Se⁺, Te⁺, As⁺, Sb⁺, P⁺ and/or Si+,
iv) controlling the temperature in the reactor to be at 0 °C to 100 °C,
v) after the reaction is finished, if necessary, purifying the reaction product.

6. Process according to claim 4 or 5, wherein the purification is performed by distillation.

7. Process according to one or more of claims 4 to 6, wherein the sulfur trioxide is provided as oleum with a sulfur trioxide content of above 0 % by weight to 65 % by weight or as pure sulfur trioxide.

8. Process according to one or more of claims 4 to 7, wherein the temperature is within a range of from above 0 °C to 70 °C, especially from 10 °C to 65 °C, preferably from 20 °C to 60 °C.

9. Process according to one or more of claims 4 to 8, wherein the cation formed *in situ* or added in step iii) reacts with the alkane to form an alkane cation,
said alkane cation reaction with SO₃ to form an alkane sulfur cation,
said alkane sulfur cation reacting again with the alkane to form the alkane sulfonic acid and again an alkane cation.

10. Process according to claim 9, wherein the alkane is methane, the alkane sulfonic acid is methane sulfonic acid, and the alkane cation is CH₃⁺.

11. A mixture comprising an alkane, sulfur trioxide, a cation being able to react with the alkane to form an alkane cation, wherein the cation is selected from a halogen cation, especially I⁺ and Br⁺, S⁺, Se⁺, Te⁺, As⁺, Sb⁺, P⁺ and/or Si⁺, and optionally a solvent.

12. A mixture comprising an alkane, sulfur trioxide, a compound forming *in situ* a cation under super acid conditions, said cation being able to react with the alkane to form an alkane cation, wherein the cation is selected from a halogen cation, especially I⁺ and Br⁺, S⁺, Se⁺, Te⁺, As⁺, Sb⁺, P⁺ and/or Si⁺, and optionally a solvent.

13. Mixture according to claim 11 or 12, wherein the solvent is sulfuric acid and/or the alkane is methane.

## Patentansprüche

1. Verwendung eines unter supersauren Bedingungen stabilen Kations als Katalysator bei der Herstellung von Alkansulfonsäuren aus Alkanen und Schwefeltrioxid, insbesondere bei der Herstellung von Methansulfonsäure aus Methan und Schwefeltrioxid, wobei das Kation mit dem Alkan unter Bildung eines Alkylkations reagieren kann, wobei Stabilität bedeutet, dass das Kation mit dem Alkan reagieren kann, sich aber innerhalb von 24 h bei Raumtemperatur (20 °C) nicht zersetzt, und wobei das Kation aus einem Halogenkation, insbesondere I⁺ und Br⁺, S⁺, Se⁺, Te⁺, As⁺, Sb⁺, P⁺ und/oder Si⁺, ausgewählt ist.

2. Verwendung nach Anspruch 1, wobei das Kation während der Herstellung von Alkansulfonsäuren unter sauren oder supersauren Bedingungen in situ gebildet wird.

3. Verwendung nach Anspruch 1, wobei das Kation vor der Zugabe zur Reaktion zum Erhalt einer Alkansulfonsäure gebildet wird.

4. Verfahren zur Herstellung von Alkansulfonsäuren, insbesondere von Methansulfonsäure, das folgende Schritte umfasst:
i) Bereitstellen von Schwefeltrioxid und einem Alkan, insbesondere Methan, in einer Reaktionskammer,
ii) Einstellen eines Drucks von 1 bis 200 bar im Reaktor,
iii) Eintragen einer in situ ein Kation bildenden Verbindung in den Reaktor, wobei das Kation aus einem Halogenkation, insbesondere I⁺ und Br⁺, S⁺, Se⁺, Te⁺, As⁺, Sb⁺, P⁺ und/oder Si⁺, ausgewählt ist,
iv) Regulieren der Temperatur im Reaktor auf 0 °C bis 100 °C,
v) gegebenenfalls Reinigen des Reaktionsprodukts nach Beendigung der Reaktion.

5. Verfahren zur Herstellung von Alkansulfonsäuren, insbesondere von Methansulfonsäure, das folgende Schritte umfasst:
i) Bereitstellen von Schwefeltrioxid und einem Alkan, insbesondere Methan, in einer Reaktionskammer,
ii) Einstellen eines Drucks von 1 bis 200 bar im Reaktor,
iii) Eintragen eines unter supersauren Bedingungen stabilen Kations in den Reaktor, wobei Stabilität bedeutet, dass das Kation mit dem Alkan reagieren kann, sich aber innerhalb von 24 h bei Raumtemperatur (20 °C) nicht zersetzt, und wobei das Kation aus einem Halogenkation, insbesondere I⁺ und Br⁺, S⁺, Se⁺, Te⁺, As⁺, Sb⁺, P⁺ und/oder Si⁺, ausgewählt ist,
iv) Regulieren der Temperatur im Reaktor auf 0 °C bis 100 °C,
v) gegebenenfalls Reinigen des Reaktionsprodukts nach Beendigung der Reaktion.

6. Verfahren nach Anspruch 4 oder 5, wobei die Reinigung durch Destillation durchgeführt wird.

7. Verfahren nach einem oder mehreren der Ansprüche 4 bis 6, wobei das Schwefeltrioxid als Oleum mit einem Schwefeltrioxidgehalt von über 0 Gew.-% bis 65 Gew.% oder als reines Schwefeltrioxid bereitgestellt wird.

8. Verfahren nach einem oder mehreren der Ansprüche 4 bis 7, wobei die Temperatur in einem Bereich von über 0 °C bis 70 °C, insbesondere von 10 °C bis 65 °C, vorzugsweise von 20 °C bis 60 °C, liegt.

9. Verfahren nach einem oder mehreren der Ansprüche 4 bis 8, wobei das in Schritt iii) in situ gebildete Kation mit dem Alkan unter Bildung eines Alkankations reagiert,
wobei das Alkankation mit SO₃ unter Bildung eines Alkanschwefelkations reagiert,
wobei das Alkanschwefelkation erneut mit dem Alkan unter Bildung der Alkansulfonsäure und erneut eines Alkankations reagiert.

10. Verfahren nach Anspruch 9, wobei es sich bei dem Alkan um Methan handelt, es sich bei der Alkansulfonsäure um Methansulfonsäure handelt und es sich bei dem Alkankation um CH₃⁺ handelt.

11. Mischung, umfassend ein Alkan, Schwefeltrioxid, ein Kation, das mit dem Alkan unter Bildung eines Alkylkations reagieren kann, wobei das Kation aus einem Halogenkation, insbesondere I⁺ und Br⁺, S⁺, Se⁺, Te⁺, As⁺, Sb⁺, P⁺ und/oder Si⁺, ausgewählt ist, und gegebenenfalls ein Lösungsmittel.

12. Mischung, umfassend ein Alkan, Schwefeltrioxid und eine unter supersauren Bedingungen in situ ein Kation bildende Verbindung, wobei das Kation mit dem Alkan unter Bildung eines Alkylkations reagieren kann, wobei das Kation aus einem Halogenkation, insbesondere I⁺ und Br⁺, S⁺, Se⁺, Te⁺, As⁺, Sb⁺, P⁺ und/oder Si⁺, ausgewählt ist, und gegebenenfalls ein Lösungsmittel.

13. Mischung nach Anspruch 11 oder 12, wobei es sich bei dem Lösungsmittel um Schwefelsäure handelt und/oder es sich bei dem Alkan um Methan handelt.

## Revendications

1. Utilisation d'un cation qui est stable dans des conditions superacides en tant que catalyseur dans la préparation d'acides alcanesulfoniques à partir d'alcanes et de trioxyde de soufre, notamment dans la préparation d'acide méthanesulfonique à partir de méthane et de trioxyde de soufre, ledit cation étant capable de réagir avec l'alcane pour former un cation alkyle, le terme « stabilité » signifiant que le cation est capable de réagir avec l'alcane mais ne se décompose pas en 24 h à température ambiante (20 °C), et le cation étant choisi parmi un cation halogène, notamment I⁺ et Br⁺, S⁺, Se⁺, Te⁺, As⁺, Sb⁺, P⁺ et/ou Si⁺.

2. Utilisation selon la revendication 1, le cation étant formé *in situ* dans des conditions acides ou superacides pendant la préparation d'acides alcanesulfoniques.

3. Utilisation selon la revendication 1, le cation étant formé avant l'ajout à la réaction pour obtenir un acide alcanesulfonique.

4. Procédé pour la préparation d'acides alcanesulfoniques, notamment d'acide méthanesulfonique, comprenant les étapes de
i) fourniture de trioxyde de soufre et d'un alcane, notamment de méthane, à un compartiment de réaction,
ii) établissement d'une pression allant de 1 à 200 bars dans le réacteur,
iii) introduction d'un composé formant *in situ* un cation dans ledit réacteur, le cation étant choisi parmi un cation halogène, notamment I⁺ et Br⁺, S⁺, Se⁺, Te⁺, As⁺, Sb⁺, P⁺ et/ou Si⁺,
iv) régulation de la température dans le réacteur pour être de 0 °C à 100 °C,
v) après que la réaction est terminée, si nécessaire, purification du produit de réaction.

5. Procédé pour la préparation d'acides alcanesulfoniques, notamment d'acide méthanesulfonique, comprenant les étapes de
i) fourniture de trioxyde de soufre et d'un alcane, notamment de méthane, à un compartiment de réaction,
ii) établissement d'une pression allant de 1 à 200 bars dans le réacteur,
iii) introduction d'un cation qui est stable dans des conditions superacides dans ledit réacteur, le terme « stabilité » signifiant que le cation est capable de réagir avec l'alcane mais ne se décompose pas en 24 h à température ambiante (20 °C), et le cation étant choisi parmi un cation halogène, notamment I⁺ et Br⁺, S⁺, Se⁺, Te⁺, As⁺, Sb⁺, P⁺ et/ou Si⁺,
iv) régulation de la température dans le réacteur pour être de 0 °C à 100 °C,
v) après que la réaction est terminée, si nécessaire, purification du produit de réaction.

6. Procédé selon la revendication 4 ou 5, la purification étant réalisée par distillation.

7. Procédé selon l'une ou plusieurs des revendications 4 à 6, le trioxyde de soufre étant fourni en tant qu'oléum doté d'une teneur en trioxyde de soufre allant de plus de 0 % en poids à 65 % en poids ou en tant que trioxyde de soufre pur.

8. Procédé selon l'une ou plusieurs des revendications 4 à 7, la température étant dans une plage allant de plus de 0 °C à 70 °C, notamment de 10 °C à 65 °C, préférablement de 20 °C à 60 °C.

9. Procédé selon l'une ou plusieurs des revendications 4 à 8, le cation formé *in situ ou* ajouté dans l'étape iii) réagissant avec l'alcane pour former un cation d'alcane,
ladite réaction de cation d'alcane avec SO₃ pour former un cation de soufre alcane,
ledit cation de soufre alcane réagissant à nouveau avec l'alcane pour former l'acide alcanesulfonique et à nouveau un cation d'alcane.

10. Procédé selon la revendication 9, l'alcane étant le méthane, l'acide alcanesulfonique étant l'acide méthanesulfonique et le cation d'alcane étant CH₃⁺.

11. Mélange comprenant un alcane, du trioxyde de soufre, un cation qui est capable de réagir avec l'alcane pour former un cation d'alcane, le cation étant choisi parmi un cation halogène, notamment I⁺ et Br⁺, S⁺, Se⁺, Te⁺, As⁺, Sb⁺, P⁺ et/ou Si⁺, et éventuellement un solvant.

12. Mélange comprenant un alcane, du trioxyde de soufre, un composé formant *in situ* un cation dans des conditions superacides, ledit cation étant capable de réagir avec l'alcane pour former un cation d'alcane, le cation étant choisi parmi un cation halogène, notamment I⁺ et Br⁺, S⁺, Se⁺, Te⁺, As⁺, Sb⁺, P⁺ et/ou Si⁺, et éventuellement un solvant.

13. Mélange selon la revendication 11 ou 12, le solvant étant l'acide sulfurique et/ou l'alcane étant le méthane.
